# DEMANDE DE BREVET EUROPEEN

(11) **EP 0 745 363 A1**
(43) Date de publication de la demande: **04.12.1996**
(21) Numéro de dépôt: 96401152.2
(22) Date de dépôt: 29.05.1996
(51) Int. Cl.: A61F 11/04

(54) **Prothèse auditive comportant un implant cochléaire à ondelettes**

(30) Priorité: 31.05.1995 FR 9506475
(71) Demandeur: BERTIN & CIE, F-78373 Plaisir Cedex (FR)
(72) Inventeur: Fadeau, Michel, Gustave, Jules, 13290 LES MILLES (FR)
(74) Mandataire: Caron, Gérard

(57) **Abrégé**

Cette prothèse auditive comporte n électrodes (e1 à en) destinées à être implantées dans la cochlée en n points différents choisis pour permettre l'identification par le cerveau d'une pluralité de bandes de fréquences différentes du spectre sonore reçu.

Un microphone (6) est connecté à des moyens (7,9) pour mesurer l'énergie instantanée du signal sonore dans les bandes de fréquences. Des moyens (3,4,15) commandent cycliquement les électrodes (e1 à en) en fonction de l'énergie mesurée dans les bandes de fréquences respectives.

Selon l'invention, la prothèse comprend également des moyens (9) pour déterminer la répartition temporelle dans le signal sonore de l'énergie dans chacune desbandes de fréquences et des moyens (14) pour exciter les électrodes (E1 à En) à chaque cycle dans l'ordre correspondant à la répartition temporelle, lesdits moyens (9) étant conçus pour appliquer une transformée d'ondelettes audit spectre sonore.

## Description

La présente invention est relative aux prothèses auditives du type comportant un implant cochléaire. De telles prothèses sont utilisées principalement pour la réhabilitation des sourds neurosensoriels disposant d'un grand nombre de fibres auditives intactes, et elles comportent à cet effet des électrodes implantées dans la rampe tympanique de la cochlée.

Les principes de ces prothèses auditives ont été décrits notamment, d'une part, dans le brevet français FR 2 383 657 pour ce qui est de leur partie technique et plus spécialement électronique, et d'autre part, dans un article de Gerald Loeb paru dans la revue "Pour la Science" en avril 1985, pages 32 à 39. Cet article illustre principalement les problèmes liés à l'implantation de l'appareil et les difficultés rencontrées au niveau du couplage entre le nerf auditif et les électrodes chargées de lui communiquer les signaux utiles. On se référera donc à ces deux documents pour plus de détails concernant l'état de la technique de ce genre de prothèses auditives.

En résumant, on peut considérer qu'à l'heure actuelle, une prothèse auditive à implant cochléaire comprend essentiellement un module externe communiquant, généralement par voie inductive, avec un module implanté dans l'oreille interne, le module externe élaborant des signaux traduisant le spectre sonore environnant pour les transmettre au module interne, accompagnés des signaux complémentaires éventuels utilisés par le module interne pour son auto-alimentation.

Le module interne comprend un groupe de n électrodes (avec à l'heure actuelle n généralement supérieur ou égal à huit), disposées en un faisceau qui est inséré dans la rampe tympanique par le chirurgien. Le module interne comprend également un circuit de commande de ces électrodes en mode bipolaire, avec, suivant les réalisations, soit une paire d'électrodes portées simultanément à des potentiels opposés, soit une seule électrode portée à un potentiel différent de l'ensemble des autres électrodes. Le module interne comprend enfin un circuit permettant d'assurer sa propre alimentation électrique, ainsi qu'une inductance sous-cutanée. Il est à noter que le nombre d'électrodes est lié principalement à la place disponible dans la rampe tympanique et aux problèmes de diaphonie entre les électrodes. Il dépend donc de la technologie de réalisation, ainsi que du protocole chirurgical d'implantation.

Le module externe de la prothèse comprend tout d'abord un microphone pour capter le spectre sonore environnant et délivrer un signal d'information sonore, un circuit de décomposition de ce signal d'information sonore en une pluralité de signaux de fréquence représentatifs de bandes de fréquences sonores prédéterminées dudit spectre sonore, des moyens permettant d'affecter à ces signaux de fréquence des paramètres relatifs, d'une part, à la physiologie générale de l'oreille et, d'autre part, aux caractéristiques particulières de l'oreille du patient et de ses électrodes implantées, et des moyens pour, à partir desdits signaux de fréquence affectés desdits paramètres, élaborer des impulsions représentatives de l'énergie contenue dans chacune desdits bandes de fréquence et destinées à être appliquées sélectivement à chacune desdites électrodes à travers une inductance communiquant avec l'inductance du module implanté.

Les électrodes sont généralement commandées de façon cyclique à l'aide d'un signal de trame qui assure une commande cyclique des électrodes pour permettre l'excitation, soit d'une électrodes à la fois, soit de deux électrodes en même temps.

Malgré le progrès technique considérable dont ont bénéficié les prothèses auditives ces derniers années sur le plan de la miniaturisation et de l'utilisation de circuits intégrés travaillant avec des logiciels spécialement adaptés notamment au niveau du filtrage du signal sonore, elles continuent à souffrir d'un certain nombre de déficiences techniques qui en rendent encore problématique une utilisation intensive dans le traitement des surdités profondes.

En effet, on constate tout d'abord une grande disparité dans les résultats obtenus parmi les patients dotés d'une prothèse, depuis un bon suivi d'une conversation sans lecture labiale par certains patients, jusqu'à une quasi absence de résultats pour d'autres, malgré une récupération certaine de l'ambiance sonore.

On sait que le signal de parole comporte un certain nombre de sons élémentaires ou phonèmes, ainsi que des régimes transitoires pour passer d'un phonème à l'autre. Ainsi, on peut distinguer d'abord les "voyelles" qui, prises au sens large, désignent un son continu produit à partir de l'excitation des cordes vocales et inclut donc des sons tels que par exemple, pour le Français, "ou", "é", "è" etc. Les voyelles sont en réalité constituées de la superposition de plusieurs fréquences appelées "formants" correspondant à la résonance des différentes cavités du conduit vocal excitées par le générateur que constituent les cordes vocales.

Il y a également les "consonnes" que l'on classe en général en différentes catégories telles que les "sifflantes", les "chuintantes", les "fricatives", etc. qui peuvent correspondre à des déplacements rapides et particuliers de la langue, à des variations rapides du débit d'air du locuteur ou encore à des réductions variables de passage d'air dans le conduit vocal.

Une consonne est donc caractérisée par un spectre de fréquences instantanées très riche et très rapidement évolutif. Cette évolution est très dépendante du son qui précède et du son qui suit.

Si on prend par exemple la prononciation du mot "CHAT" par un locuteur français, la consonne "CH" donne lieu à un spectre de fréquences très riche comparable à celui d'un bruit blanc ou encore du "souffle" ambiant de l'air dans le microphone, alors que la voyelle "A" se traduit par deux fréquences relativement bien définies modulées par la fréquence fondamentale des cordes vocales.

On comprend ainsi que, du fait de l'extrême complexité des spectres sonores instantanés, l'une des difficultés majeures que l'on rencontre dans la conception des prothèses auditives à implant cochléaire réside dans la transcription la plus fidèlement possible du spectre sonore instantané reçu, en des signaux de fréquence utilisables pour être adaptés à la commande appropriée des électrodes du module implanté de la prothèse.

Or, les solutions préconisées à cet égard dans l'art antérieur ne sont pas satisfaisantes essentiellement pour les deux raisons suivantes en fonction de la façon dont l'analyse spectrale est réalisée.

D'une part, les filtres électroniques utilisés pour la décomposition du spectre sonore ne travaillent pas assez rapidement, car ils nécessitent un certain temps pour réagir au signal d'entrée qui entre dans leur bande passante et également ils s'amortissent progressivement, lorsque la fréquence du signal d'entrée sort de la bande passante du filtre. Il en résulte que la représentation fréquentielle instantanée qu'ils produisent n'est pas fidèle d'autant que dans la pratique, l'exploitation d'un banc de filtres pour une prothèse à implant cochléaire nécessite généralement un multiplexage de lecture des sorties des différents filtres, rajoutant encore de l'imprécision à la détection d'apparition de telle ou telle fréquence.

D'autre part, dans les réalisations plus récentes pour lesquelles on met généralement en oeuvre une décomposition par transformée de Fourier rapide ou FFT, on prend en compte une fenêtre de temps limitée et établit en conséquence le spectre moyen sur cette fenêtre, indépendamment des conditions d'évolution du spectre du signal d'entrée pendant cette fenêtre temporelle.

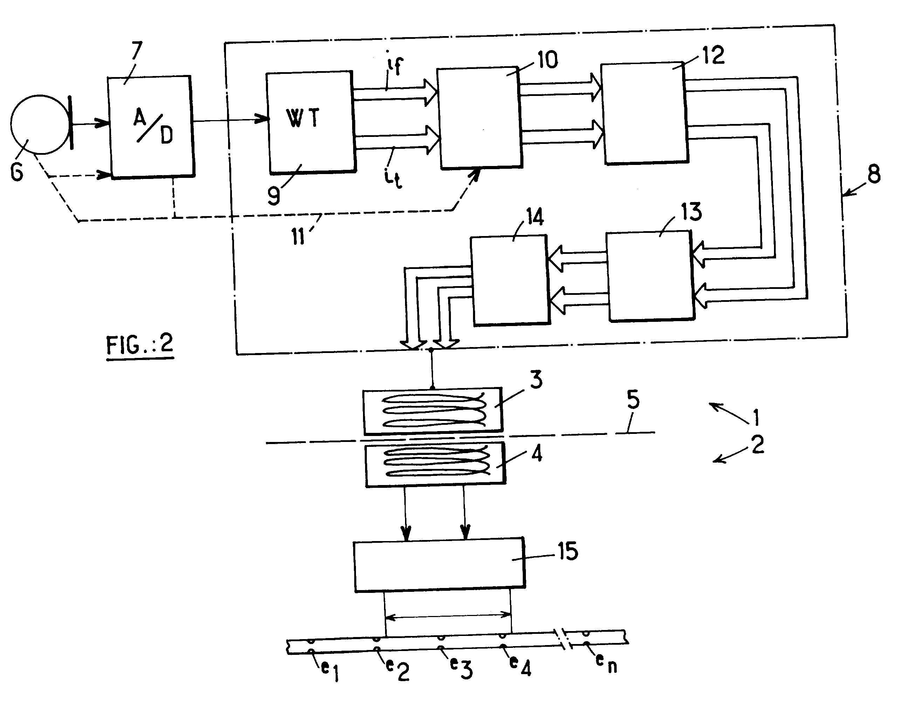

On a récemment développé des techniques de décomposition de signaux utilisant ce que l'on dénomme communément la "théorie des ondelettes". Cette technique a été décrite dans un article de O. Rioul et M. Vetterli dans la revue "IEEE SP Magazine", paru dans le numéro d'octobre 1991, pages 14 à 38. Cet article met en évidence, notamment par sa figure 5.4, qu'un spectre sonore peut être décomposée en ses composantes de fréquence par la "transformée d'ondelettes" avec une finesse temporelle d'autant plus grande que la fréquence dans le spectre est plus élevée. Ceci revient à dire que les signaux obtenus en sortie d'un système d'analyse mettant en oeuvre la transformée d'ondelettes, peuvent contenir non seulement une information d'énergie par fréquence ou par bande de fréquences, mais également une information temporelle par fréquence ou par bande de fréquences.

Ainsi, l'invention a pour but de proposer une prothèse auditive du type décrit ci-dessus, permettant de fournir à l'oreille interne une information sonore correspondant plus fidèlement au spectre sonore instantané appliqué à la prothèse et fournir ainsi au patient qui en est équipé, sinon un certain confort d'écoute reproduisant au mieux celui d'un bien entendant, du moins une meilleure perception du spectre sonore instantané qui l'entoure.

L'invention a donc pour objet une prothèse auditive du type comportant n électrodes destinées à être implantées dans la cochlée respectivement en n points différents choisis pour permettre l'identification par le cerveau d'une pluralité de bandes de fréquences différentes du spectre sonore reçu par la prothèse, celle-ci comprenant:
- un microphone,
- des moyens pour mesurer l'énergie instantanée du signal sonore dans lesdites bandes de fréquences, et
- des moyens pour commander cycliquement lesdites n électrodes en fonction de l'énergie mesurée dans lesdites bandes de fréquences respectives,
- caractérisée en ce qu'elle comprend également
- des moyens pour déterminer la répartition temporelle dans le signal sonore de l'énergie dans chacune desdites bandes de fréquences, et
- des moyens pour exciter lesdites électrodes à chaque cycle dans l'ordre correspondant à ladite répartition temporelle.

Il résulte de ces caractéristiques, que les électrodes réparties dans la rampe tympanique de l'oreille peuvent être commandées, non seulement en fonction de l'énergie qu'elles doivent recevoir, énergie qui est propre à la bande de fréquences à laquelle telle ou telle électrode est affectée, mais également en fonction d'une information temporelle permettant d'adapter les instants de leur excitation aux phénomènes temporels contenus dans le spectre sonore reçu.

Suivant une autre caractéristique de l'invention, lesdits moyens pour déterminer la relation temporelle sont conçus pour appliquer une transformée d'ondelettes audit spectre sonore.

D'autres caractéristiques et avantages de la présente invention apparaîtront au cours de la description qui va suivre, donnée uniquement à titre d'exemple et faite en se référant aux dessins annexés sur lesquels:
- la figures 1a et 1b sont des diagrammes fréquence/temps du spectre sonore apparaissant lorsqu'un locuteur français prononce le mot "ALICE";
- la figure 2 est un schéma de principe d'une prothèse auditive selon l'invention; et
- la figure 3a à 3c montrent des diagrammes illustrant en fonction du temps, le fonctionnement de la prothèse auditive selon l'invention.

On a représenté sur la figure 2 le schéma simplifié d'un exemple de réalisation d'une prothèse auditive selon l'invention.

Dans la configuration représentée, cette prothèse comprend un module externe 1 et un module interne ou implantable 2, la communication entre ces deux modules étant réalisée par l'intermédiaire d'une inductance émettrice 3 située à l'extérieur et d'une inductance réceptrice 4 implantée sous la peau 5 du patient.

Le module externe 1 comprend un microphone 6 porté par le patient de manière à pouvoir capter le spectre sonore environnant. La sortie de ce microphone est raccordée à un convertisseur analogique/numérique 7, ce convertisseur travaillant, de préférence à une fréquence d'échantillonnage relativement élevée (au moins de l'ordre de 8 kHz, par exemple). Bien entendu, on peut prévoir entre ce microphone 6 et le convertisseur 7 un dispositif de réglage de volume automatique ou manuel (non représenté).

La sortie du convertisseur 7 est raccordée à un microprocesseur 8 du type processeur de traitement de signal, par exemple avec ses circuits intégrés périphériques (mémoires, séquenceur, etc. Ce microprocesseur matérialise par son programme interne les moyens permettant de réaliser les fonctions qui vont maintenant être décrites et qui sont représentées symboliquement sur la figure 2 à l'aide de rectangles appropriés. L'homme de l'art sera à même d'écrire le programme adéquat pour faire fonctionner le microprocesseur 8 à l'aide des éléments de description donnés ci-après de ces fonctions.

Le signal numérique élaboré par le convertisseur 7 est d'abord soumis en 9 à des moyens d'analyse par transformée d'ondelettes selon les préceptes exposées dans l'article précité de MM Rioul et Vetterli. Cette analyse donne lieu à la production d'essentiellement deux informations If et It dans chaque bande de fréquences examinée du spectre sonore, à savoir d'une part, une information fréquentielle traduisant le contenu énergétique de la bande de fréquence examinée et, d'autre part, une information temporelle traduisant la position relative dans le temps des informations énergétiques des bandes de fréquences les unes par rapport aux autres.

Il est à noter que les moyens d'analyse par transformée d'ondelettes utilisent une fenêtre temporelle de durée variable en fonction de la fréquence et que, selon une caractéristique avantageuse de l'invention, cette fenêtre temporelle d'analyse est amenée à se déplacer en continu dans le temps, les résultats de l'analyse obtenus à un temps donné subissant le traitement ultérieur dans le microprocesseur 8 et étant transmis au module 2, pendant qu'une nouvelle analyse est faite en 9 dans la fenêtre d'analyse suivante.

Cette façon de travailler introduit, certes, un retard dans la transmission des éléments du spectre sonore au nerf auditif du patient, mais ce retard reste sensiblement constant et faible et n'est donc pas à même de perturber le déroulement d'une conversation. Le retard peut être de l'ordre de quelques dixièmes de seconde au maximum, ce qui est comparable aux retards éventuellement rencontrés sur certaines liaisons téléphoniques à grande distance par satellite.

Les informations fréquentielle If et temporelle It sont ensuite soumises en 10 à un traitement dit des "stationnarités" permettant d'identifier et éventuellement de supprimer dans l'information transmise le bruit ambiant du spectre sonore.

Cette identification peut éventuellement être facilitée en prévoyant un microphone auxiliaire (non représenté) dont les signaux, après numérisation, sont appliqués au microprocesseur 8, liaison qui est symbolisée sur la figure 2 par les traits en pointillés 11. Par ailleurs, la mise en oeuvre de cette fonction peut être rendue réglable de manière que le patient puisse lui-même en commander l'application à l'information transmise à l'oreille et éventuellement améliorer ainsi le rapport signal/bruit par suppression des fréquences appartenant au bruit ambiant.

Les moyens symbolisés par le rectangle 12 mettent en oeuvre l'application des données du modèle général de l'oreille interne relevé sur un sujet bien entendant, aux informations tant fréquentielle que temporelle élaborées par la mise en application des fonctions 9 et 10. Ces données correspondent au résultat d'un transcodage instantané entre les niveaux sonores correspondant à chacune des bandes de fréquence analysées et les impulsions électriques théoriquement mesurables sur les sites correspondants de la cochlée d'un bien entendant. Ainsi, grâce à l'invention, ce modèle peut prendre en compte non seulement la tonotopie de la cochlée, c'est-à-dire la correspondance entre différents points de la cochlée et les différentes fréquences constituant la plage des sons audibles, mais également le comportement temporel de la cochlée, c'est-à-dire par exemple les décalages temporels rencontrés dans la transmission sur le nerf auditif d'un bon entendant des différentes composantes fréquentielles instantanées d'un signal sonore, en particulier de courte durée.

La fonction symbolisée par le rectangle 13 représente l'application de données analogues relatives au modèle personnalisé des électrodes implantées qu'il est éventuellement possible d'observer chez le patient porteur de la prothèse.

Il est à noter que les moyens 12 et 13 peuvent être mis en oeuvre par exemple en stockant dans la mémoire du microprocesseur des tables d'équivalence permettant d'affecter aux données instantanées relatives à chaque bande élémentaire de fréquences, des corrections d'amplitude, de fréquence et d'occurrence dans le temps.

Des moyens de commande 14 également matérialisés par le microprocesseur 8 sont destinés à mettre en forme les informations fréquentielle et temporelle, éventuellement corrigées par les paramètres élaborés par les moyens 12 et 13, pour qu'elles puissent être transmises aux électrodes e1, e2, ..... en disposées dans la rampe tympanique du patient. Ces moyens de commande permettent ainsi d'élaborer pour chaque électrode un signal d'énergie regroupant les informations relatives à l'énergie, la récurrence et la synchronisation, cette dernière information représentant le rapport temporel entre le signal relatif à une électrode donnée, et ceux des autres électrodes.

Les moyens de commande 14 du module externe 1 peuvent être conçus essentiellement selon deux modes de mise en oeuvre.

Ils peuvent engendrer un train d'impulsions répétitif destiné à être appliqué sous la forme d'une trame cyclique aux électrodes el à en par l'intermédiaire de l'émetteur 3, et des organes du module implantable 2, c'est-à-dire le récepteur 4 et le circuit de commande 15. Ce dernier assure également l'alimentation électrique des électrodes et éventuellement des autres circuits du module interne 2, à partir de l'énergie transmise à travers l'émetteur 3 et le récepteur 4.

Chaque trame comprend alors une information relative au rapport entre la fréquence de récurrence de l'impulsion sur l'électrode concernée et la fréquence de la trame de transmission. La trame peut également comprendre une information concernant l'écart de synchronisme entre l'impulsion considérée et une référence temporelle, cette dernière pouvant être identique pour toutes les électrodes. Par exemple la référence temporelle peut être le début de la première impulsion de chaque trame.

Ces différentes informations peuvent être transmises par combinaison de différentes modulations du signal transmis, par exemple par modulation d'amplitude, de fréquence et/ou de phase.

L'autre mode de mise en oeuvre peut consister à engendrer une succession de mots binaires contenant chacun le numéro d'ordre de l'électrode, l'amplitude de l'impulsion à appliquer, la fréquence de récurrence des impulsions pour chaque électrode et/ou l'écart de synchronisation par rapport à la référence.

Le circuit de commande 15 situés dans le module implantable 2 est conçu pour décoder l'ensemble des informations qui lui parvient à travers l'émetteur 3 et le récepteur 4. Il produit donc les séquences de commande des électrodes selon les ordres qui lui arrivent par l'intermédiaire des moyens de commande 15.

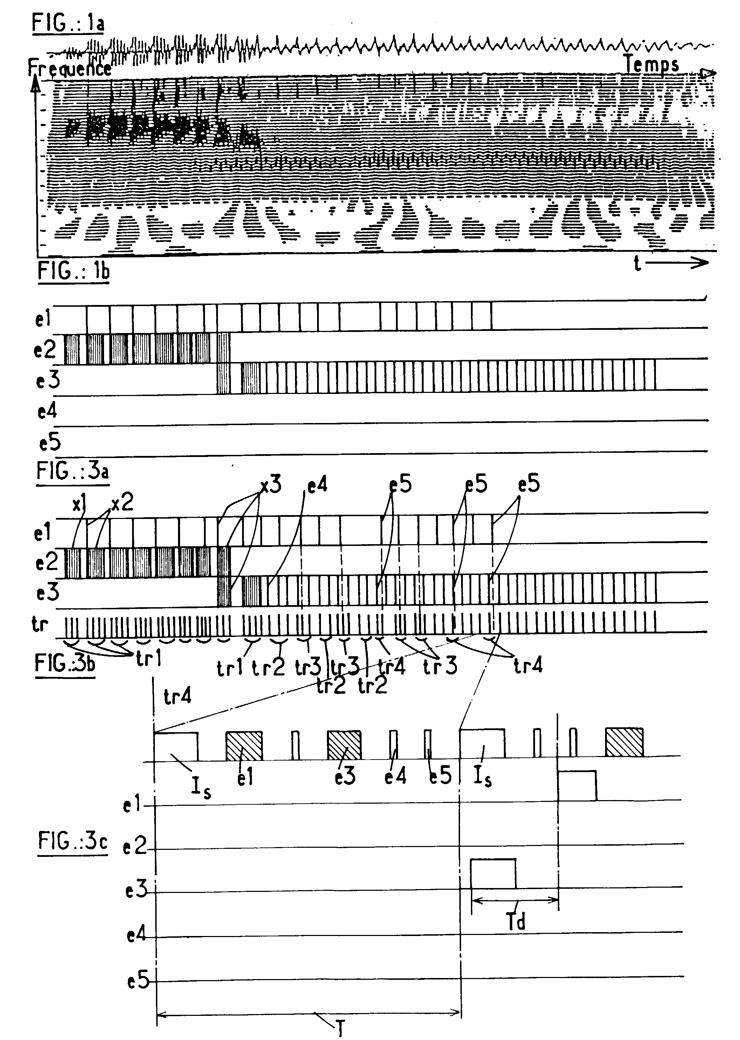

Les figures 3a à 3c illustrent le fonctionnement de la prothèse selon l'invention dans le cas où les électrodes reçoivent leurs impulsions de commande moyennant la modulation en fréquence d'une onde porteuse qui est transmise de l'émetteur 3 au récepteur 4. Pour simplifier ces figures, on a supposé que la prothèse ne comporte que cinq paires d'électrodes el à e5. Par ailleurs, l'exemple porte sur la perception du mot "ALICE" prononcé par un locuteur français et dont la figure la montre en fonction du temps t, la forme d'onde issue du microphone 6. Le diagramme de la figure 1b en montre l'analyse par transformée d'ondelettes faite sur un grand nombre de points d'échantillonnage et sur 57 voies, à l'aide de fenêtres temporelles variables avec la fréquence. Le diagramme de la figure 1b fait apparaître très clairement le contenu en énergie des diverses bandes de fréquences (la fréquence est ici portée en ordonnées). Par exemple, la voyelle "A" au début de la prononciation du mot, montre une succession de "tâches noires" qui sont dues à cette voyelle.

Comme déjà indiqué, on suppose que la prothèse comprend cinq électrodes affectées respectivement à cinq bandes de fréquences correspondantes. Le diagramme de la figure 3a représente la répartition de ces bandes de fréquences parmi les électrodes e1 à e5, la bande des plus hautes fréquences étant affectée à l'électrode e1 et la bande des plus basses fréquences à l'électrode e5.

On remarquera par ailleurs que pour restituer le mot "ALICE", on considère que les deux bandes de fréquences inférieures ne contiennent pas d'information révélatrice et qu'il n'est pas nécessaire de transmettre cette information vers les électrodes e4 et e5. Par conséquent, sur la figure 3b, les lignes correspondant aux électrodes e4 et e5 sont vides.

La figure 3b ne montre donc plus que les signaux correspondant aux électrodes significatives e1, e2 et e3. En outre, on a représenté symboliquement à la ligne désignée par "tr", la synchronisation des trames dans lesquelles les informations destinées aux électrodes sont transmises à ces dernières. Pour le besoin de la représentation, chaque trame est ainsi symbolisée par un seul bâtonnet.

Au début de l'exemple, si on considère le premier élément d'information x1, on voit que trois trames trl sont nécessaire pour le transcrire, la première trame étant synchronisée sur le début de x1. Ces trames sont régulièrement espacées dans le temps et ne concernent encore que l'électrode e2. Le deuxième élément d'information x2 comporte une parcelle pour l'électrode el et une parcelle pour l'électrode e2. Cet élément peut être transcrit par quatre trames tr1 régulièrement espacées et synchronisées sur le début de l'élément x2. La transcription se poursuit ainsi par des trames tr1 jusqu'à ce que l'information devienne moins dense. On notera que juste avant la fin de cette phase (élément x3), les trois électrodes e1, e2 et e3 sont toutes concernées.

A partir de l'élément x4, la densité de l'information à transmettre devient plus faible. On peut alors se contenter d'espacer les trames (désormais désignées par tr2), le début de chaque trame ou groupe de trames restant synchronisé sur l'apparition de l'élément d'information de l'électrode concernée.

Les trames tr3 correspondent alternativement à des informations affectées aux électrodes e1 et e3. Elles apparaissent donc au gré de l'apparition de ces informations et sont synchronisées sur celles-ci.

Les éléments d'information x5 concernent également deux électrodes e1 et e3, mais dans ce cas ils sont espacés l'un de l'autre d'une durée inférieure à celle d'une trame. Mais ces trames tr4 ont ceci de particulier que l'information affectée à l'électrode el apparaît plus tard que celle de l'électrode e3. Cette relation temporelle, prise en compte grâce à l'invention et en particulier au processus d'analyse utilisant la transformée d'ondelettes, peut être utilisée pour commander les électrodes concernées.

C'est ce que représente, à une échelle de temps agrandie, la figure 3c qui montre le déroulement du processus de commande de ces électrodes. Ce diagramme fait d'abord apparaître la dernière trame tr4 de la figure 3b. On voit que la trame commence par une impulsion de synchronisation Is suivie de cinq impulsions relatives respectivement aux électrodes e1 à e5. On notera que chaque trame comporte toujours une information d'électrode pour chacune d'entre elles afin de pouvoir situer dans le temps l'information utile relative à chaque électrode. Ainsi dans la trame représentée, l'information relative à l'électrode e1 est significative et correspond à une impulsion dont la largeur traduit l'énergie devant être transmise à cette électrode. En revanche, l'électrode e2 n'est pas concernée pendant cette trame, qui contient de ce fait seulement une courte impulsion d'ordre relative à cette électrode. Puis, l'information destinée à l'électrode e3 est également représentée par une impulsion d'énergie, tandis que les électrodes e4 et e5 ne sont représentées que par leur impulsion d'ordre.

On comprendra que chaque trame (que ce soit cette trame tr4 spécifique ou les autres trames trl, tr2 ou tr3) est ainsi transmise entre l'émetteur 3 et le récepteur 4 accompagnée d'une information de phase qui représente la relation temporelle requise entre les impulsions destinées respectivement aux électrodes el et e3 (décalage Td et ordre d'apparition).

C'est ainsi que dans l'exemple de la figure 3c, les électrodes el à en sont commandées avec un certain retard, fonction du temps de calcul requis par le microprocesseur, par rapport au début de la trame concernée. D'après l'information d'ordre (non représentée) mentionnée ci-dessus, associée à la trame tr4, l'électrode e3 doit être excitée avant l'électrode el. L'électrode e3 est donc excitée en premier et l'électrode el 'est avec le retard Td mentionné ci-dessus par rapport à l'électrode e3.

La transmission est réalisée, par exemple au moyen d'une porteuse dont la modulation tout ou rien en amplitude correspond aux impulsions d'énergie et qui peut être modulée en fréquence ou en phase par exemple pour ce qui concerne la relation temporelle entre les informations destinées aux diverses électrodes. Le décodage d'une telle porteuse modulée est réalisé dans le circuit de commande 15 incorporé dans le module interne 2.

On comprendra que la transmission des informations vers ce circuit et le décodage se font avec un très léger écart temporel par rapport à la production du contenu de chaque trame, comme on le voit d'ailleurs sur le diagramme e) de la figure 3. Toutefois, la préparation d'une trame donnée peut se faire pendant que la trame préparée précédemment est transmise et décodée de sorte que la perception du spectre sonore ne subit aucune discontinuité.

## Revendications

1. Prothèse auditive du type comportant n électrodes (e1 à en) destinées à être implantées dans la cochlée en n points différents choisis pour permettre l'identification par le cerveau d'une pluralité de bandes de fréquences différentes du spectre sonore reçu, cette prothèse comprenant:
- un microphone (6),
- des moyens (7, 9) pour mesurer l'énergie instantanée du signal sonore dans lesdites bandes de fréquences,
- des moyens (3, 4, 15) pour commander cycliquement lesdites n électrodes (e1 à en) en fonction de l'énergie mesurée dans les bandes de fréquences respectives,
ladite prothèse étant caractérisée en ce qu'elle comprend également:
- des moyens (9) pour déterminer la répartition temporelle dans le signal sonore de l'énergie dans chacune desdites bandes de fréquences,
- des moyens (14) pour exciter lesdites électrodes (E1 à En) à chaque cycle dans l'ordre correspondant à ladite répartition temporelle.

2. Prothèse suivant la revendication 1, caractérisée en ce que lesdits moyens pour déterminer ladite répartition temporelle (9) sont conçus pour appliquer une transformée d'ondelettes audit spectre sonore.

3. Prothèse suivant l'une quelconque des revendications 1 et 2, caractérisée en ce que lesdits moyens (14) pour exciter lesdites électrodes sont conçus pour engendrer des trains d'impulsions successives constituant une trame (tr1 à tr4) dans laquelle lesdites impulsions sont définies en fonction de ladite énergie et de ladite relation temporelle.

4. Prothèse suivant la revendication 3 caractérisée en ce que lesdits moyens (14) pour exciter lesdites électrodes sont conçus pour définir lesdites impulsions par leur durée, leur fréquence de récurrence et/ou leur phase relative.

5. Prothèse suivant la revendication 4, caractérisée en ce que ladite relation temporelle est définie par rapport à une référence temporelle telle que le début de la première impulsion de chacune desdites trames (tr1 à tr4).

6. Prothèse suivant l'une quelconque des revendications 1 et 2, caractérisée en ce que lesdits moyens (14) pour exciter lesdites électrodes sont conçus pour engendrer une succession de mots binaires dans lesquels sont contenues les informations d'ordre de commande desdites électrodes, d'amplitude des impulsions à leur appliquer et/ou d'énergie devant être contenue dans lesdites impulsions.

7. Prothèse suivant l'une quelconque des revendications précédentes, caractérisée en ce qu'elle comprend également des moyens (12, 13) pour affecter à l'information d'énergie dans lesdites bandes de fréquences au moins un paramètre traduisant le comportement physiologique de l'oreille d'un bien entendant et/ou des électrodes implantées dans l'oreille interne du patient.

8. Prothèse suivant l'une quelconque des revendications précédentes, caractérise en ce qu'elle comprend en outre des moyens (10) pour affecter à l'information d'énergie dans lesdites bandes de fréquences au moins un paramètre prenant en compte le bruit ambiant.
